# EUROPEAN PATENT APPLICATION

(11) **EP 1 471 376 A1**
(43) Date of publication of application: **27.10.2004**
(21) Application number: 02806644.7
(22) Date of filing: 30.09.2002
(51) Int. Cl.: G02C 13/00, G02C 7/04, G06F 17/60

(54) **CONTACT LENS AND EXAMINATION SYSTEM**

(30) Priority: 01.02.2002 JP 2002026120
(71) Applicant: Menicon Co., Ltd., Nagoya-shi, Aichi-ken 460-0006 (JP)
(72) Inventor: TSUSHI, Yasuaki, c/o MENICON CO., LTD., Nagoya-shi, Aichi 460-0006 (JP); ISHIHARA, Akira, c/o MENICON CO., LTD., Nagoya-shi, Aichi 460-0006 (JP)
(74) Representative: Senior, Alan Murray
(86) International application number: PCT/JP2002/010202
(87) International publication number: WO 2003/065111

(57) **Abstract**

Provides a novel supply and examination system by which it is possible to safely provide users with contact lenses having a predetermined expiration date, without imposing an excessive burden on users and on ophthalmologists. A user 18 is assessed a periodic membership fee on the one hand, while the user 18 is supplied with contact lenses through a designated pre-selected merchant 14. That is, member users 18 who pay a periodic membership fee on a continuing basis are supplied with new replacement contact lenses without any special procedure, simply by visiting the merchant 14. When receiving supply of lenses, user 18 received periodic eye examinations at the merchant 14. Various kinds of information including personal user information are managed by the contact lens supplying entity 10 to build a database 24.

## Description

### TECHNICAL FIELD

The present invention relates to a novel supply and examination system for contact lenses, by means of which is possible for users to safely and securely use contact lenses having finite recommended expiration dates (scheduled replacement date), such as daily, weekly, biweekly, or monthly. The present invention is also concerned with a novel contact lens supply and examination method.

### BACKGROUND ART

Contact lenses have been used for vision correction in the case of myopia, hyperopia, astigmatism, presbyopia and the like, and are available in a wide variety of designs and materials. An increase in the number of contact lens users in recent years has been accompanied by growth in the number of retail outlets that handle contact lenses, and the ways in which contact lenses are distributed for sale have become more diverse.

Incidentally, since contact lenses are medical devices, when a user intends to purchase contact lenses, it is important to determine so by deciding whether or not the use of contact lenses is appropriate for the user, and then selecting contact lenses with the proper shape and optical characteristics for the particular user.

Such decisions and determinations when purchasing contact lenses should properly be made by an ophthalmologist having experience and specialized knowledge with regard to contact lenses. Generally, results of past ophthalmologic examinations of a user, information regarding prior experience with contact lens wear, and the like constitute effective data when deciding whether contact lenses are suitable for a user or when selecting an appropriate contact lens. Therefore, such information relating to contact lenses for an individual user should be collected and kept.

In recent years, there has been a tendency for contact lenses to be handled like any typical mass consumption goods. As a result of the increase in the number of wearers of conventional hard type and soft type contact lenses, and the growth in sales of so-called disposable contact lenses having very short expiration dates, this trend towards "mass consumption goods" of contact lenses has accelerated. Not only contact lens retailers but ophthalmologists as well are already having difficulty in coping with the increase in customers, and the increasing diversity of customer flows associated with the increase in retail outlets, and of the contact lenses themselves. Thus, the state of affairs of customer contact lens date collection and record keeping at retail outlets and ophthalmologist offices has become increasingly impracticable.

As regards ophthalmologic examination of the user when purchasing contact lenses as well, the trend towards "mass consumption goods" of contact lenses has resulted in a significant degree of variation in levels of professional knowledge and experience. This, in association with the aforesaid fact that collection and record keeping of user information is often poor, poses the risk that a user may not receive an appropriate examination and proper contact lens selection.

With this state of affairs, in consideration of supply of contact lenses such as the "disposable" products mentioned above, which have finite scheduled replacement dates (expiration dates) that are daily, weekly, biweekly, or monthly, and use of same by users, there is a risk of users experiencing significant problems due, for example, to:
① using a contact lens unsuited to the user, due to the ease with which contact lenses may be obtained;
② the user continuing to use contact lenses past their expiration date; or
③ the user failing to obtain a proper examination, diagnosis, or treatment. Indeed, actual incidents of this kind have been reported.

Possible strategies for addressing such problems could include, for example:
(a) ensuring that users acknowledge full responsibility for managing their own care; and
(b) ensuring that retailers who supply contact lenses to users implement through user management.

However, with regard to (a), it is not difficult to imagine that this approach will be largely ineffective. With regard to (b), there variations in examination and diagnostic capabilities among retailers, and not only is it impractical to impose the burden of user management on an extremely large number of retailers, but the largest problem is that a user does not necessarily always purchase contact lenses from the same retailer.

Accordingly, in contact lens supply systems to date, it was not possible when supplying disposable contact lenses to fully guarantee user safety, and even at present, despite considerable time having passed since large numbers of users actually made the switch from non-disposable, long term wear contact lenses to disposable contact lenses having specified expiration dates, the reason that Menicon K.K. (the Applicant) has not actively attempted to scale up supply or advertise such disposable contact lens products lies in this inability to adequately ensure user safety, which is one of the most paramount considerations.

With the foregoing in view, it is an object of the present invention to provide a novel supply and examination system for contact lenses, as well as a novel contact lens supply and examination method, by means of which is possible to safely supply to users contact lenses having predetermined expiration dates, while enabling users to easily obtain supply of and use such contact lenses.

### DISCLOSURE OF THE INVENTION

The modes of the invention for the purpose of addressing the problems are disclosed hereinbelow. Elements employed in the several modes disclosed herein may be employed in arbitrary combination with one another where it is possible to do so. The modes and technical features of the invention are not limited to those disclosed herein, and will be understood to include other inventive ideas derived from the disclosure in the specification as whole and the drawings, or apparent to those who skilled in the art from the disclosure.

One aspect of the invention relating to a supply and examination system for contact lenses adapted to supply to users contact lenses that have predetermined finite scheduled replacement date, and to examine the users, comprising: (a) a server device for use by a supply entity for the contact lenses; (b) memory means utilizable by the server device; (c) a plurality of merchant client devices for respective use by a plurality of system merchants able to provide eye exams, the merchant client devices transmitting personal information consisting of a combination of exam information obtained from an eye exam of each of the users and supply information regarding contact lenses supplied to each of the users, together with identifying information for each of the users to the server device via communication network means, for storing the personal information in the memory means; (d) membership fee payment status monitoring means for inputting to the server device payment information regarding periodically recurring membership fees paid by each of the users to the supply entity for the contact lenses, and storing the payment information in association with the identifying information for the each of the users in the memory means; and (e) lens supply authorizing means that, in response to a request from each of the merchant client devices transmits a contact lens supply authorization signal through judgment based on the personal information and the membership fee payment information for each of the users, from the server device to the each of the merchant client devices through the communication network means.

The operation and effects of a contact lens supply and examination system structured according to the invention will be described.

In the first instance, the first major feature lies in the fact that user management is realized by means of a contact lens supply entity, rather than self-management by users or user management by stores that retail contact lenses to users. That is, by having the contact lens supply entity carry out information management, there is no need to impose a burdensome workload on individual users or on small retail stores, while enabling vast amounts of information to be managed efficiently overall. Further, by enabling a contact lens supply entity to survey, assess, or test merchants in order to select affiliate merchants for supplying contact lenses to users, it becomes possible to ensure that the ability to fit users with appropriate contact lenses and carry out examinations is maintained at or above a certain level. Additionally, in the event that a user moves, has a job transfer, or in some instances goes on a business trip, it is possible for the user to request to use a location (merchant) different from the usual one.

The second major feature lies in the fact that users are assessed a periodically recurring membership fee. That is, by being assessed a periodically recurring membership fee, a user is able to receive supply of new contact lenses when the expiration data is reached. It is important that this membership fee is assessed periodically, it being a notable feature that contact lens purchase cost is not simply prepaid or paid on a per-transaction basis. That is, insofar as a membership fee is assessed, the user will perceive that he or she is taking a loss unless he or she receives supply of contact lenses when the expiration date is reached, and it may be expected that this perception can be utilized to induce the user, without any particular perception of being compelled to do so, but rather with a perception of taking voluntary action, to proceed to his or her merchant to receive supply of new contact lenses, at which time the user will receive an eye exam at the merchant is question. In short, the system of the present invention by means of designing a specific physical system that skillfully utilizes the psychology of the user, is able to realize with a fair level of probability a situation in which in the case of so-called disposable contact lenses having expiration dates, users are prompted to periodically replace their contact lenses and receive periodic eye exams, in which feature resides significant technical significance, and hence the inventive step, of the present invention. In the event that a user fails to make periodic payment, it would of course be possible, for example, once it has been verified that the user has failed to receive supply of new lenses despite the scheduled replacement date having been reached, to then take some countermeasure such as canceling the contact lens supply agreement with the user, once a predetermined grace period has elapsed.

In order for a contact lens supply authorization signal to be issued by lens supply authorizing means, as a general rule, it is a precondition that the expiration date of contact lenses previously supplied to the user has been approximately reached, and that moreover the user proceed to his or her merchant and receive an eye exam. Of course, it would be possible also to issue a contact lens supply authorization signal on the precondition of some other predetermined reason, such as damage or loss, even if the contact lens expiration date has not been reached.

A third major feature lies in the fact that it becomes possible to ensure product quality of disposable lenses. That is, by designing a system according to the present invention, the contact lens supplying entity enjoys, by way of a secondary benefit accompanying ensuring of user safety, the benefit of to a certain extent "locking in" the user, who is also a customer. As a result, somewhat more consistent profitability will result, whereby, reductively it becomes possible to aim at improving contact lens product quality, while holding down the monetary burden to the user, as compared to conventional disposable contact lenses supplied by one-time purchases or bulk purchases.

In respect of the fact that system merchants in the invention must be capable of carrying out eye exams it is typically necessary, for example in Japan as of the time of this writing, that an ophthalmologist be present on-site. However, it is not necessary that the ophthalmologist and the retail outlet (store) that actually supplies the user with the contact lenses be the same entity. For example, the ophthalmologist and the retail outlet could exist independently, exist in a relationship, exist in affiliation, or have some other substantially unitary existence in a certain physical or legal form, such various modes being understood to be included in the term "system merchants."

Following are a number of exemplary preferred modes for contact lens supply and examination systems structured according to the invention.

Specifically, in a contact lens supply and examination system of the invention, it is advantageous to adopt an arrangement wherein in the lens supply authorizing means the decision as to whether each of the users has received periodic eye exams is made on the basis of exam information for each of the users stored in the memory means, and the contact lens supply authorization signal is transmitted the contact lens supply authorization signal to each of the merchant client devices on the condition that the periodic eye exams have been received.

Further, in a contact lens supply and examination system of the invention, it is advantageous to adopt an arrangement that includes a plurality of user client devices for use respectively by a plurality of the users and able to receive supply of information from the server device, the arrangement being such that by transmitting an information disclosure request signal to the server device through the communication network, and on the condition of confirmation of correspondence of each of the users by means of the identifying information or the like, each of the users is able to be provided by the server device with personal information, the membership fee payment information, and the like stored in the memory means in relation to each of the users.

Further, in a contact lens supply and examination system pertaining to the invention, it is advantageous to adopt an arrangement that includes a plurality of user client devices for use respectively by a plurality of the users and able to receive supply of information from the server device, each being arranged such that on the basis of personal information for each of the users and the membership fee payment information stored in the memory means, a scheduled replacement date for the contact lenses is transmitted from the server device to each of the user client devices, on the condition that the periodic membership fee has been paid.

Further, in a contact lens supply and examination system pertaining to the invention, it is advantageous to adopt an embodiment with an arrangement that includes a rewritable card provided to each of the users and having required personal information stored thereon; and card processing devices provided for respective use by the system merchants, able to read the personal information stored on the rewritable card, and rewrite the personal information if necessary.

The contact lens supply and examination method according to the invention, on the other hand, features a method for providing contact lenses to a user and examining the user, wherein (α) in response to a plurality of system merchants able to provide eye exams, each transmitting personal information consisting of a combination of exam information obtained from an eye exam of each of the users and supply information regarding contact lenses supplied to each of the users, together with identifying information for each of the users, via communication network means to a server device for use by a supply entity for the contact lenses, (β) the personal information is stored in memory means utilizable by the server device, (γ) payment information of periodic membership fee made from each of the users to the contact lens supply entity is stored in the memory means, (δ) at the time of the finite scheduled replacement date of the contact lenses, each of the system merchants, upon verification of periodic membership fee payment information, provides each of the users coming to the store with an eye exam and with a supply of new contact lenses, and (ε) in response to each of the system merchants transmitting information regarding the exam conducted on each of the uses and contact lenses supply information, together with identifying information for the each of the users, via the communication network means to the server device, (ζ) the personal information for each of the users stored in the memory means is updated, and the contact lens supply entity resupplies each of the system merchants with contact lenses for restocking.

According to this method of the present invention, the advantage of being able to safely provide users with contact lenses having predetermined expiration dates, as well as enabling users to readily receive supply of contact lenses, may be effectively realized, as will be understood from the description of the system of the present invention described previously.

### BRIEF DESCRIPTION OF DRAWINGS

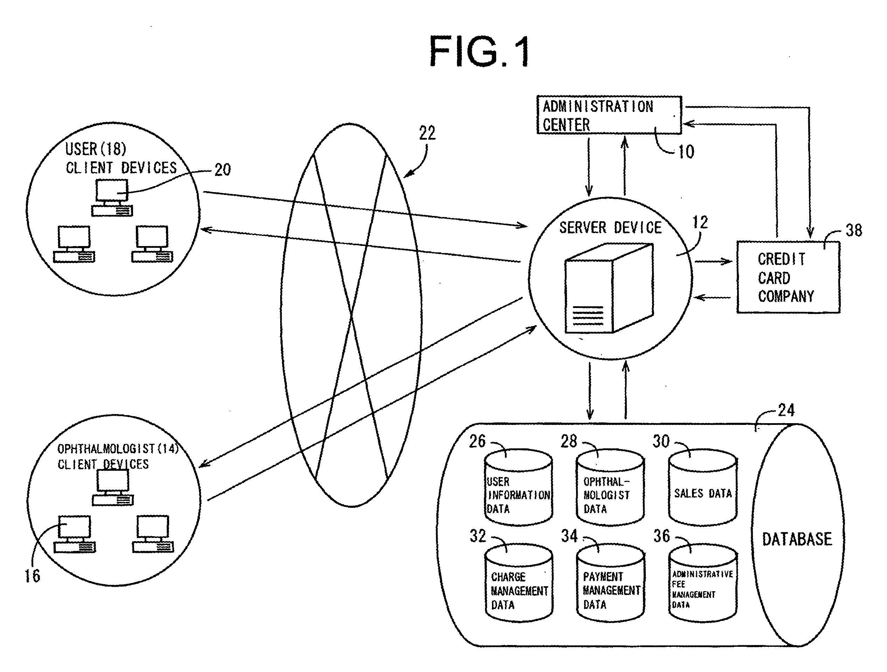

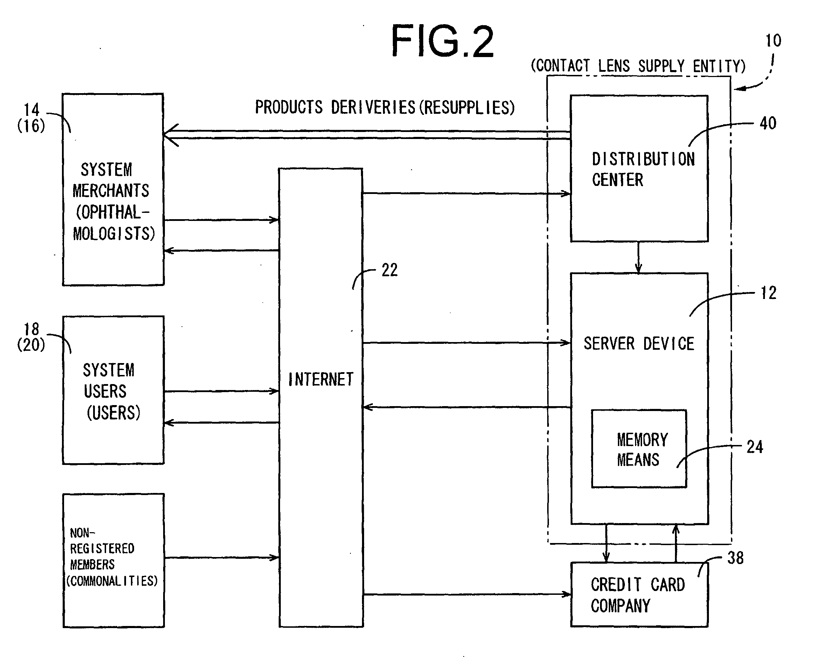

Fig. 1 is a schematic illustration of one embodiment of a hardware arrangement for working a contact lens sales method according to the invention. Fig. 2 is a block diagram describing one specific process mode when executing a contact lens sales method according to the invention, using the system depicted in Fig. 1.

### BEST MODE FOR CARRYING OUT THE INVENTION

In order to further specifically clarify the invention, there will be described the embodiments of the invention with reference to the accompanying drawings.

Referring first to Fig. 1, there is shown an overall schematic illustration of a structure of one embodiment of a contact lens supply and examination system arranged according to the invention, suitable for working the contact lens supply and examination method according to the invention. The sales system of this embodiment is under the administration of an administration center 10 which is part of the contact lens supply entity, and comprises (a) a server device 12 for use by the contact lens supply entity; (b) a database 24 serving as memory means utilizable by the server device 12; (c) a plurality of ophthalmologist client devices 16 as merchant client devices for use by a plurality of ophthalmologists 14 who are system merchants able to provide eye exams, the devices being used to transmit via communication network means 22 to the server device 12 a combination of exam information obtained from an eye exam of a user and supply information regarding contact lenses supplied to the user, together with identifying information for the user, by way of personal information for storage in the database 24 serving as the aforementioned memory means; (d) membership fee payment status monitoring means 38, 12 etc. for inputting to the server device 12 payment information regarding the periodically recurring membership fee paid to the contact lens supply entity by each user, and creating a record of this information in association with identifying information for users in the memory means 24 (34); and (e) lens supply authorizing means 12, 24 that, in response to a request from an ophthalmologist client device 16 transmits a contact lens supply authorization signal, based on personal information and membership fee payment information for the user, from the server device 12 to the ophthalmologist client device 16 through the communication network means 22.

The server device 12, ophthalmologist client devices 16 and user client devices 20 may each be composed of a computer comprising a keyboard, mouse or other input device, and a CRT, liquid crystal display or other display device, so as to enable sending and receiving of information via the Internet. In particular, the server device 12 is configured as a WWW server for providing Web pages over the Internet 22, while the ophthalmologist client devices 16 and the user client devices 20 have a WWW browser installed to enable viewing of Web pages over the Internet. Additionally installed on the server device 12, the ophthalmologist client devices 16 and user client devices 20 are suitable software enabling sending and receiving of information of various kinds over the Internet using e-mail or other means and, optionally, software for encoding and decoding sent and received signals.

The server device 12 basically constitutes a single unit, whereas the ophthalmologist client devices 16 and user client devices 20 are each provided in large numbers. In particular user client devices 20 are appropriately constituted as personal computers, mobile terminal computers, cell phones, PHS or other devices capable of connecting to the Internet 22 to access Web pages posted on the Internet 22, in order to be suitably constituted as these personal computers, mobile terminal computers, cell phones, PHS or other devices. Ophthalmologist client devices 16 are provided only to merchants having knowledge, experience, decision making ability or the like at or above certain predetermined levels, that can be substantially verified to be possessed of an ophthalmologist who can be demonstrated to have continuously and consistently provided professional diagnoses relating to contact lenses or to include such an ophthalmologist, and, optionally, on the condition of a having a predetermined contractual agreement with the administration center 10 (contact lens supply entity). It is determined in advance with reference to population or the like, so that the ophthalmologist client devices 16 are dispersed at appropriate distances apart throughout the entire sales service area.

The sales system of this embodiment further comprises a database 24 as memory means administered and utilized by the server device 12. Besides a database in the narrow sense, database 24 may be provided as a computer-administered storage device of any of various kinds equipped with a suitable storage medium such as RAM, CD-R, CD-RW, DVD-R, DVD-RAM, MO, MD, PD, HD etc., and may store data in a file format that can be administered directly by the server device 12. Where the database 24 in the narrow sense is employed, the server device 12 and database 24 will be arranged in a database system including suitable schema commonly known in the art.

The database 24 is configured so as to contain user information data 26, ophthalmologist data 28, sales data 30, charge management data 32, payment management data 34, and administrative fee management data 36.

The user information data 26 represent stored personal information for a user who has received an eye exam from the ophthalmologist 14, and includes (a) specific information for the individual user, (b) diagnosis information (including examination and checkup information, etc.), and (c) user identifying information. The (a) specific information for the individual user may include data such as, for example, user name, address, date of birth, age, sex, phone number, occupation, employer/school, or e-mail address, as well as credit card number and expiration date, or survey information. It may also include, in relation to associated diagnosis information, a prescription date, prescription expiry period, number identifying the hospital (eye clinic) issuing the prescription or number identifying the physician (ophthalmologist). The (b) diagnosis information represents diagnosis information such as prescription, etc. issued in the diagnosis by the ophthalmologist 14, and by clearly indicating, for example, the contact lens type, base curve, power (diopter), DIA (diameter), contains enough information to specifically identify a contact lens suitable for the user. In addition, there may optionally be included examination information relating to the condition of the user's eyes, such as whether the condition of contact lens wear is satisfactory, or whether there are any special symptoms of note. The (c) user identifying information is information associated with each user for the purpose of identifying users, and optionally may include, for example, the user's birth date or other specific password, or an ID issued each user and sent to the user by the ophthalmologist 14 during diagnosis by the ophthalmologist 14, for example.

The ophthalmologist data 28 store individual information for merchants in the sales system, that requires the ophthalmologist 14, and may store, for example, the individual name of each ophthalmologist belonging to an merchant; his or her affiliated hospital; name and address of the hospital; date of registration in the system; number of staff; telephone number; store ID code; diagnosed user-associated information; number of associated users; and number of records of diagnosis information on the server device 12, as well as survey information, e-mail address, or other data.

The sales data 30 stores information regarding contact lenses sold according to the sales system of this embodiment, and may store, for example, type, product name, and specifications (base curve, power, diameter) of contact lenses supplied to a user, corrected vision provided by the supplied contact lenses, uncorrected vision, axis of astigmatism, magnitude of astigmatism, added magnitude, supply date, supply method, and other data, as well information such as expiration date or replacement date for the contact lenses, the information being stored in associated with the user.

The charge management data 32 store charge information for payments made by users who have purchased contact lenses, method of payment on a user-by-user basis, and other such data. The payment management data 34 store credited payment information for users who have purchased contact lenses, credit method, and other data. The administrative fee management data 36 store data relating to administrative fees for data entry, which are paid to ophthalmologists as compensation for requesting the ophthalmologists 14 to assume the duties of reporting ophthalmologic diagnosis information relating to contact lens adaptability in relation to users and to suitable contact lenses. By means of transmitting this information from ophthalmologist client devices 16 for storage in the database 24, the fee management data may include for each individual ophthalmologist the amount paid, payee, payment method, payment date, payment history, and the like.

The sales system of this embodiment comprises a credit card company 38 as one constituent element of the system. As the credit card company 38, an existing company different from the product supplying entity may be utilized. The credit card company 38 basically contracts on an individual basis with the user 18, for example, paying a certain amount set on the basis of the contract to the product supplying entity (administration center 10) as payment for contact lenses, and debiting the user 18 for an amount equivalent to the amount so paid. By incorporating a credit card company 38 into the sales system in this way, the labor required on the part of the product supplying entity (administration center 10) in terms of investigating the ability of users to pay when sending contact lenses to users prior to payment, collecting payment, and so on can be reduced. Also, with this system users periodic pay a membership fee at a given interval of, for example, every month, every few weeks, or every few months. Collection of membership fees at periodic intervals is carried out by the product supplying entity (administration center 10), utilizing the credit card company 38.

In a sales system constituted in the above manner, when purchasing contact lenses, the user 18 receives an examination by the ophthalmologist 14 at a merchant, and after registration of the required items in the database 24 has been executed, is supplied directly by the merchant with contact lenses having an expiration date.

The entire process that takes place when a new user 18 utilizes the sales system of this embodiment to procure contact lenses, up to the point that registration of the required items in the database 24 is received, shall be described in brief hereinbelow with reference to the block diagram in Fig. 2.

First, the new user 18 connects to the Internet 22 using the user client device 20, such as a personal computer or cell phone, searches for and views a Web page provided by the server device 12 of administration center 10, and looks for the ophthalmologist 14 as a merchant affiliated with the sales system of this embodiment. The search for the ophthalmologist 14 may be carried out, for example, using e-mail or the like from the Web page, by means of the user 18 sending from the user client device 20 to the server device 12, by way of criteria for selecting the ophthalmologist 14, preferences such as a preferred location or train station, examination time, and so on, whereupon the server device 12 introduces the user 18 via e-mail several ophthalmologists 14 who generally meet the preference information, thereby improving convenience for the user 18.

The user 18 then visits an appropriate selected ophthalmologist 14, and receives an examination from the ophthalmologist 14. The ophthalmologist 14 examines the user 18 and prepares a medical report (chart) and diagnosis information indicating a contact lens prescription. This diagnosis information consists of information indicating the contact lens type (material, construction etc.) and specifications (base curve, power, diameter) suitable for the user, and may be prepared in the form of a prescription. In this embodiment, the following description assumes that diagnosis information is provided as a prescription.

Next, the ophthalmologist 14 transmits, for storage in the database 24 of server device 12, the contents of the diagnosis information (prescription) prepared on the basis of the examination of the user 18, his or her (i.e. the ophthalmologist's 14) identification number (No.), and a prescription identification number (No.). This storage operation is carried out by inputting the data to the ophthalmologist client device 16 connected to server device 12 via the Internet 22, and transmitting the data to the server device 12.

In response to receipt of the aforementioned diagnosis information, eye clinic No., and prescription No., the server device 12 initially retransmits the information received from the ophthalmologist client device 16 back to the ophthalmologist client device 16 via the Internet 22, for confirmation by the ophthalmologist 14. Input errors in crucial diagnosis information and the like may be avoided thereby. Where necessary, the server device 12 may receive an information content verification result signal transmitted from the ophthalmologist client device 16, so that the result of information content verification may be verified without error in the server device 12.

The server device 12 then creates a record in the database 24 serving as the memory means, containing the user diagnosis information, eye clinic No., and prescription No. sent to the server device 12 by the ophthalmologist client device 16, and then terminates the process. When creating a record of information in the database 24, in order for diagnosis information, eye clinic No., and prescription No. associated with each user 18 to be placed in a record, information associated with user 18 may be created in server device 12 and placed in a record in the database 24 simultaneously with the diagnosis information, eye clinic No., and prescription No. After the information has been stored in the database 24, optionally, payment of an information input administrative fee may be made by the administration center 10 to the ophthalmologist 14 who has performed the information recording process with ophthalmologist client device 16. Additionally, once recording of information of various kinds relating to the user has been completed in the above-described manner, the user 18 may be assigned unique user identifying information (ID) by the server device 12, sent by e-mail or by post.

In conjunction with registration of diagnosis information etc. for the use in database 24, member registration of user 18 and a process to register the user 18 in the membership fee payment system are executed. In this embodiment, the user 18 pays membership fees by means of a credit card company in which he or she is already enrolled.

For example, at the merchant with which the ophthalmologist 14 is affiliated, the user 18 enrolls in a contract with a credit card company if necessary, and a credit check is performed. This check is performed from the ophthalmologist client device 16 at the merchant, by transmitting required information pertaining to the user 18 to the server device 12 or credit card company 38, to check the authenticity of the credit card information, validity of the credit card and, optionally, any history of use of the sales system by the user in the past; and for the credit card-related items, transmitting data from the server device 12 to the credit card company 38 via a dedicated line, to make request to the credit card company 38. As a result of the check, if there appears to be a significant problem that would result in denial of membership registration, a membership denial check result is sent, together with the reason, by the server device 12 to the ophthalmologist client device 16, whereupon the process terminates. In this case, information for the denied user 18 may be recorded and stored in the database 24, together with the result of the check.

If there is no problem with the check results, the server device 12 send a contact lens supply authorization signal to the ophthalmologist client device 16, whereupon the process terminates. On the condition of having received such a contact lens supply authorization signal, the merchant affiliated with the ophthalmologist 14 registers in the database 24 the diagnosis information etc. for the user 18 who has received the examination, and then supplies contact lenses appropriate for the user 18, typically with same-day service. In preferred practice, each merchant will have on hand a stock of appropriate quantities of supplied contact lenses.

The merchant affiliated with the ophthalmologist 14 not only supplies contact lenses to user 18, but also formally registers the user 18 as a member, and well as registering information relating to supply of contact lenses to the user. Registration is carried out from the ophthalmologist client device 16 through transmission to server device 12 via the Internet 22. The server device 12 receives the signal, performs member registration of the user 18 for the purpose of being provided with contact lenses having expiration dates, creates fee request data for requesting the user 18 for a membership fee assessed to the user 18, and transmitting this fee request data from the server device 12 to the credit card company 38 over a dedicated line, to request the credit card company 38 for periodic collection of a membership fee for supply of contact lenses.

Process information that includes the fact of supply of contact lenses to the user 18 by the ophthalmologist 14, information and specifics thereof, and request data transmission to the credit card company 38 is recorded in the database 24, whereupon the server device 12 transmits to the administration center 10 lens an instruction containing ordering information, so that the merchant affiliated with the ophthalmologist 14 supplying the lenses is restocked with the specific contact lenses provided to the user 18. Upon receiving this instruction, the administration center 10 sends to a distribution center 40 having the contact lenses in stock an instruction to ship the contact lenses, and in accordance with this instruction the distribution center 40 ships the contact lenses to the merchant, so that the merchant's stock of contact lenses is maintained.

Together with shipping the contact lenses to the merchant, the administration center 10 adjusts the system so that required information recorded on the server can be supplied in response from the merchant affiliated with the ophthalmologist 14, through a client device 16. Optionally, an additional information input administration fee may be paid by the administration center 10 who is the supplying entity, to the merchant affiliated with the ophthalmologist 14 as compensation for cooperation. With this, the series of processes for supplying contact lenses to a user is complete.

Of the information held in memory in the database 24, the server device 12 searches user information data on a periodic (e.g. daily or every several days) basis, and selects from among a multiplicity of users 18 supplied with contact lenses those whose contact lens replacement date (expiration date) is coming up within a predetermined period of time, for example, within one week. Client devices 20 of the selected users 18 having received supply of contact lenses are then notified via the Internet 22 that the replacement date is approaching. On the basis of this information, the user 18 is prompted to soon revisit the merchant affiliated with the ophthalmologist 14, and to thereby receive supplied of new replacement contact lenses.

Here, the user 18 who has come to the merchant's store at the time of the replacement date is supplied with replacement contact lenses on the condition of receiving an eye exam and diagnosis from the ophthalmologist 14. At this time, the merchant may verify information regarding whether the user 18 is a qualified member, whether membership fees have been paid on a continuous basis, and the like, by requesting such information from the server device 12 or, optionally, the credit card company 38, from the ophthalmologist's client device 16 via the Internet 22, and having received a contact lens supply authorization signal from the server device 12, to supply the user 18 with new replacement contact lenses, to supply the user 18 with novel replacement contact lenses in consideration of the results of the examination as well.

Subsequently, the merchant sends information indicating that new contact lenses have been supplied to the user 18, information regarding the result of the user's 18 examination, and so on to the server device 12 from the ophthalmologist's client device 16 via the Internet 22. The server device 12 receiving this information creates, modifies or updates, as necessary, the information in the database 24.

As a general rule, the number of contact lenses supplied by the ophthalmologist 14 to a user will be several lenses for each required eye. However, this number is not limited, it being possible in the case where certain circumstances exist, in consideration of these circumstances and in consideration of the results of an examination of the user 18 or the like to instead supply lenses on at a time for each required eye, so as encourage a prompt follow-up examination a subsequent time, or to instead supply a greater number of lenses for each required eye than is the general rule, leaving it up to the user to determine the appropriate number of replacement lenses to be supplied with when the expiration date arrives. Thus, it becomes possible to extend the interval before the next examination to a longer time interval than is the general rule, there being no need for an ophthalmologist to prohibit any such mode which may be taken at his or her own initiative. Specifically, for example, in the case of a contact lens having an expiration date of one month, it would be possible as a general rule to provide the user 18 with a three-month supply for each eye at one time, at the time of each examination.

Accordingly, by means of the contact lens sales system according to the present invention as set forth hereinabove, by means of using a computer system that includes the Internet 22, it becomes possible to efficiently build a sales system architecture and to carry out a contact lens supply operation utilizing the sales system, without imposing an excessive burden on the users 18 and the ophthalmologists 14, and without the need for large staffing requirements at the administration center 10. Thus, the users 18, without any particular perception of being compelled to do so, but rather on the basis of a perception of taking voluntary action, may be prompted to proceed to the merchant to receive supply of new contact lenses when the expiration date has been reached, at which time the user receives an eye exam at the merchant in question, so that even in the case of so-called disposable contact lenses having an expiration date, there may be realized with a fair level of establishment periodic replacement of contact lenses by the user, and periodic examination of the user, whereby safe and comfortable contact lens wear may be advantageously achieved.

In short, the user 18 is assessed a periodic membership fee on the one hand, while the user 18 is supplied with contact lenses by a specific pre-selected merchant 14, whereby the member user 18 who is paying the periodic membership fee on a continuing basis may be targeted for supply of new replacement contact lenses without any special procedure, simply by visiting the merchant 14. Further, when receiving supply of contact lenses, the user 18 receives periodic examinations at the merchant 14.

In this system, by means of employing a special system design, large amounts of data, including contact lens power, fit, and the like, for each user 18 can be stored and accumulated in the database 24 on a continuing basis as historical data over an extended period of time, automatically without the need for any special processing, whereby users, ophthalmologists and, optionally, other organizations may utilize it to readily obtain important data that can be used for reference. That is, information of various kinds including personal information for users is administered by the contact lens supplying entity 10 so as to build the database 24, whereby there can be effectively realized an efficient and extremely significant data architecture.

While the invention has been shown in detail hereinabove through a specific embodiment, this is merely exemplary, and the invention should in no way be construed as being limited to the specific disclosure of the embodiment hereinabove.

For example, whereas in the illustrated embodiment there has been described an example of a case wherein payment of membership fees by the user 18 was made by credit card, some other method of payment of membership fees would be possible, such as using direct payment in cash or the like. Payment of membership fees by member users 18 using a convenience store, post office, of other existing financial or money transfer system widely distributed throughout different areas of the country would be possible as well.

Additionally, it would of course be possible to supply some kind of service by way of a benefit supplied to member users 18 paying the periodic membership fee. In this case, for example, it would be possible to replace contact lenses at no cost or at reduced cost in the case that contact lenses are lost or damaged before their expiration date, due to some kind of accident. As the mode used for replacing contact lenses, for example, an upper limit could be established for the number of contact lenses replaced within a predetermined period, or there could be established a required time interval from the time that contact lenses are once replaced, until the next time that they will be replaced. Alternatively, when replacing contact lenses, it would be possible provide member users 18 who have not received such replacement service for a predetermined period of time preceding with complementary spare contact lenses, for example, by being allowed to replace them before the expiration date, or otherwise provide some service the use of which may be left up to the discretion of the user 18.

Furthermore, as a service for supplying contact lenses to users having purchased same using this sales system, it would be possible, for example, to periodically or non-periodically transmit information relating to contact lenses or the like from the server device 12 to user client devices 20 by means of e-mail or the like; or to execute a service such as periodic vision testing by an ophthalmologist 14 or the like, storing the results thereof in database 24, which could assist the user 18 or ophthalmologist 14 in understanding and managing the user's eyesight or the like.

Further, there could be provided a service limited to users who purchase contact lenses using the sales system herein, whereby they are entitled to consultation with administration center 10 by means of an interview, by e-mail, or the like, thereby further improving service and achieving enhanced management information.

Furthermore, by way of one support measure provided to ophthalmologists who cooperate with the sales system, it would be possible to design a referral system for referring merchants including ophthalmologists to users, whereby an ophthalmologist's office location, hours of operation, exam times, telephone number and other such information may be provided by e-mail in response to a request by a registered member, or to provide cooperating ophthalmologists, either directly or through a registered member, with ophthalmologic data such as contact lenses, eyesight, etc. of the user, which information has been accumulated in the database 24 of the sales system.

The sales system may be limited to providing service to specific members, whereby it becomes possible readily achieve a higher level of member understanding and information management, and to enhance service content. As a possible membership services mode, it would be possible for example to register members on the condition of meeting some predetermined check provision by a credit card company or the like, to recognize users on the condition of continued payment of a membership fee that recurs on a periodic, e.g. monthly, basis, and to enable utilization of the aforementioned contact lens sales system by these recognized users only; and as a supplemental service, to provide member users with periodic free vision testing by a designated ophthalmologist, or to provide free contact lens replacement or exchange, or to set up specific contact lens or care product retailers as service points in order to provide a higher level of service.

While not given individually herein, the present invention may be reduced to practice in various other modes incorporating variations, modifications and improvements which would be apparent to those skilled in the art, and these embodiments will of course fall within the scope of the invention insofar as they do not depart from the spirit thereof.

### INDUSTRIAL APPLICABILITY

As will be apparent from the preceding description, according to the present invention, it is now possible to supply medical devices, namely contact lenses of expiration-dated type, on the condition of periodic eye examination and lens replacement at periodic intervals, without imposing an excessive burden in terms of labor and time on users and on ophthalmologists, and with a perception on the part of the user of spontaneously taking voluntary action, whereby so-called disposable contact lenses that current pose a high risk can be used more safely by users.

Further, according to the present invention, utilizing a special computer system designed to include communication network means, information about contact lens use and history, eyesight, and other ophthalmologic information for individual users can be efficiently collected and kept over extended periods of time without imposing an excessive burden on users and on ophthalmologists; and on the basis of information obtained in this manner, more specialized, higher levels of support may be provided to contact lens users and to ophthalmologists, thereby providing also a significant effect in promoting proper use of contact lenses.

## Claims

1. A supply and examination system for contact lenses adapted to supply to users of contact lenses that have predetermined finite scheduled replacement date, and to examine the users comprising:
a server device for use by a supply entity for the contact lenses;
memory means utilizable by the server device;
a plurality of merchant client devices for respective use by a plurality of system merchants able to provide eye exams, the merchant client devices transmitting personal information consisting of a combination of exam information obtained from an eye exam of each of the users and supply information regarding contact lenses supplied to each of the users, together with identifying information for each of the users to the server device via communication network means, for storing the personal information in the memory means;
membership fee payment status monitoring means for inputting to the server device payment information regarding periodically recurring membership fees paid by each of the users to the supply entity for the contact lenses, and storing the payment information in association with the identifying information for the each of the users in the memory means; and
lens supply authorizing means that, in response to a request from each of the merchant client devices transmits a contact lens supply authorization signal through judgment based on the personal information and the membership fee payment information for each of the users, from the server device to the each of the merchant client devices through the communication network means.

2. A supply and examination system for contact lenses according to claim 1, wherein in the lens supply authorizing means the decision as to whether each of the users has received periodic eye exams is made on the basis of exam information for each of the users, stored in the memory means, and the contact lens supply authorization signal is transmitted the contact lens supply authorization signal to each of the merchant client devices on the condition that the periodic eye exams have been received.

3. A supply and examination system for contact lenses according to claim 1 or 2, further comprising a plurality of user client devices for use respectively by the users and able to receive supply of information from the server device, each being arranged such that by transmitting an information disclosure request signal to the server device through the communication network, and on the condition of confirmation of correspondence of each of the users by means of the identifying information or the like, the each of the users is able to be provided by the server device with personal information, the membership fee payment information, and the like stored in the memory means in relation to each of the users.

4. A supply and examination system for contact lenses according to any one of claims 1-3, further comprising a plurality of user client devices for use respectively by a plurality of the users and able to receive supply of information from the server device, each being arranged such that on the basis of personal information for each of the users and the membership fee payment information stored in the memory means, a scheduled replacement date for the contact lenses is transmitted from the server device to each of the user client devices, on the condition that the periodic membership fee has been paid.

5. A supply and examination system for contact lenses according to any one of claims 1-4 further comprising a rewritable card provided to each of the users and having required personal information stored thereon; and card processing devices provided for respective use by the system merchants, able to read the personal information stored on the rewritable card, and rewrite the personal information if necessary.

6. A method for providing contact lenses having a predetermined finite expiration date to users and examining the users, wherein
in response to a plurality of system merchants able to provide eye exams, each transmitting personal information consisting of a combination of exam information obtained from an eye exam of each of the users and supply information regarding contact lenses supplied to each of the users, together with identifying information for each of the users, via communication network means to a server device for use by a supply entity for the contact lenses,
the personal information is stored in memory means utilizable by the server device,
payment information of periodic membership fee made from each of the users to the contact lens supply entity is stored in the memory means,
at the time of the finite scheduled replacement date of the contact lenses, each of the system merchants, upon verification of periodic membership fee payment information, provides each of the users coming to the store with an eye exam and with a supply of new contact lenses, and
in response to each of the system merchants transmitting information regarding the exam conducted on each of the uses and contact lenses supply information, together with identifying information for the each of the users, via the communication network means to the server device,
the personal information for each of the users stored in the memory means is updated, and the contact lens supply entity resupplies each of the system merchants with contact lenses for restocking.
